(19) Europäisches Patentamt
European Patent Office
Office européen des brevets

(11) Numéro de publication: **0 035 429**
**B1**

(12) # FASCICULE DE BREVET EUROPEEN

(45) Date de publication du fascicule du brevet: **12.06.85**

(51) Int. Cl.⁴: **A 61 K 39/02,** A 61 K 39/385

(21) Numéro de dépôt: **81400258.0**

(22) Date de dépôt: **19.02.81**

(54) **Complexe vaccinal contenant un antigène spécifique et vaccin le contenant.**

(30) Priorité: **20.02.80 FR 8003707**

(43) Date de publication de la demande:
**09.09.81 Bulletin 81/36**

(45) Mention de la délivrance du brevet:
**12.06.85 Bulletin 85/24**

(84) Etats contractants désignés:
**AT BE CH DE FR GB IT LI LU NL SE**

(56) Documents cités:
**FR - A - 2 253 499**
**FR - A - 2 305 990**
**FR - A - 2 360 314**
**FR - A - 2 374 911**
**FR - A - 2 388 563**

**Arzneim.-Forsch./Drug Res., 30(I), Nr. 1 a (1980) DE L. DUSSOURD D'HINTERLAND: "Introduction to the Study of Ribosomal Vaccines"**
**Arzneim.-Forsch./Drug Res., 30 (I), Nr. 1a, 142-172 (1980) DE R. FONTANGES et al.: "Study of the Immunogenicity of Ribosomes and Ribosomal RNA extracted from K. pneumoniae and S. pneumoniae"**

(73) Titulaire: **PIERRE FABRE S.A., 125, rue de la Faisanderie, F-75116 Paris (FR)**

(72) Inventeur: **Dussourd d'Hinterland, Lucien, Dr., Domaine de Plombières Avenue de Lautrec, F-81100 Castres (FR)**
Inventeur: **Normier, Gérard, 23, rue Francis Poullenc, F-81100 Castres (FR)**
Inventeur: **Pinel, Anne-Marie, 22, rue des Capucins, F-81100 Castres (FR)**
Inventeur: **Durand, Jacques, 6, rue du Bearn, F-81100 Castres (FR)**

(74) Mandataire: **Corre, Jacques Denis Paul et al, Cabinet Regimbeau 26, Avenue Kléber, F-75116 Paris (FR)**

EP 0 035 429 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention, réalisée au Centre d'Immunologie et de Biologie Pierre FABRE, concerne des complexes d'antigènes bactériens spécifiques couplés à des ARN ou à des fragments d'ARN ribosomaux provenant des mêmes bactéries ou de bactéries différences, leur procédé de préparation et des vaccins les contenant.

Fontanges et al. (Arzneimittel Forschung, 30 (I) 142–172, 1980) ont étudié le pouvoir vaccinant d'ARN ribosomaux bactériens contaminés par la présence de polysaccharides ou de lipopolysaccharides.

Pour un grand nombre de bactéries, il est connu que le pouvoir vaccinant est lié à la présence d'antigènes spécifiques.

Ce sont presque toujours des antigènes de surface présents dans les parois, les membranes, les capsules, ou libérés sous forme soluble dans le milieu de culture. Leur nature est essentiellement polysaccharidique, polypeptidique ou glycoprotéique. Ces antigènes sont bien connus et leurs méthodes de préparation ont été largement décrites dans la littérature.

L'utilisation directe, en tant que vaccins, de ces antigènes spécifiques de sérotype, pour séduisante qu'elle soit, n'est pas toujours sans inconvénients. Le pouvoir antigénique de ces substances est souvent modeste, ce qui conduit à l'administration de doses élevées pour obtenir une protection significative. Cependant, à de telles doses, ces antigènes ne sont pas toujours dépourvus de toxicité, Par exemple dans le cas des polysaccharides capsulaires, ceux-ci sont en général impliqués dans la pathogénicité des bactéries et l'utilisation de doses vaccinantes élevées présente de graves inconvénients.

Or, il a été observé que des ARN ribosomaux ou des fragments de ces mêmes ARN pouvaient être avantageusement utilisés comme transporteurs actifs de ces antigènes spécifiques avec lesquels ils forment un complexe particulièrement immunogénique à très faibles doses, dû semble-t-il à une structure spatiale directement utilisable par les cellules immunocopétentes de l'organisme.

C'est pourquoi la présente invention concerne un complexe vaccinal caractérisé en ce qu'il est constitué par des ARN ribosomaux bactériens ou des fragments d'ARN ribosomaux bactériens sur lesquels on a couplé de 1 à 5% en poids d'un antigène spécifique de sérotype bactérien.

Selon l'invention, les ARN ribosomaux et l'antigène spécifique peuvent provenir des mêmes souches bactériennes ou de souches différentes.

Par «couplage» on entend de façon générale au moins une liaison entre les deux types de molécule, liaison qui est de préférence covalente par réaction des différentes fonctions des molécules en présence, mais qui peut être de nature différente.

Les ARN ribosomaux utilisables peuvent, en particulier, être extraits des ribosomes des souches suivantes:

- Streptococcus pneumoniae,
- Streptococcus pyogenes,
- Staphylococcus aureus,
- Klebsiella pneumoniae,
- Serratia marcescens,
- Escherichia coli,
- Salmonella typhimurium,
- Corynebacterium (parvum, acnès, granulosum),
- Mycobacterium (tuberculosis, Smegmatis).

Mais, il a été remarqué que l'ARN ribosomal pouvait être avantageusement remplacé par des fragments d'ARN de poids moléculaire inférieur, obtenus notamment par hydrolyse ménagée de ce même ARN ribosomal.

Cette hydrolyse peut être notamment réalisée par des méthodes classiques chimiques ou enzymatiques à l'aide de ribonucléases. Le poids moléculaire des fragments obtenus est compris entre $3 \times 10^4$ et $2 \times 10^5$ daltons et on utilise de préférence des poids moléculaires entre 30 000 et 50 000.

Parmi les antigènes spécifiques utilisables il faut citer:

- les polysaccharides capsulaires notamment de:
  Klebsiella pneumoniae
  Streptococcus pneumoniae
  Hemophilus influenzae
  Escherichia coli;
- les lipopolysaccharides membranaires des bactéries gram négatives, notamment:
  Klebsiella pneumoniae
  Serratia marcescens
  Escherichia coli
  Neisseria meningitidis
  Salmonella typhimurium;
- les protéines membranaires spécifiques, notamment:
  Escherichia coli
  Serratia marcescens
  Salmonella typhimurium
  Streptococcus pyogenes;
- les acides teichoïques et lipoteichoïques notamment de:
  Streptocoques
  Staphylocoques
  Lactobaciles.

Selon leur nature chimique et leur localisation en surface de la bactérie, ou dans le milieu de culture, ces antigènes spécifiques sont préparés suivant les procédés décrits dans l'art antérieur.

La formation d'un complexe de très faibles quantités de ces antigènes avec des ARN ou des fragments d'ARN ribosomaux, permet en administrant des doses dépourvues de toute toxicité d'obtenir un haut niveau de protection spécifique et, élargir ainsi considérablement les possibilités d'application.

Pour ce qui concerne les ARN ribosomaux totaux ou leurs fragments ils peuvent être préparés par des procédés connus.

Un exemple de préparation d'ARN ribosomal sera donné en exemple, car il s'agit là d'une préparation semi-industrielle ou industrielle des ARN ribosomaux. Ce procédé comporte essentiellement la préparation des ribosomes bactériens par précipitation fractionnée à partir des bactéries broyées, solubilisation des protéines ribosomales par le SDS à chaud, précipitation de l'ARN qui est ensuite traité par la PRONASE pour éliminer les protéines résiduelles, puis l'ARN obtenu est à nouveau purifié par chromatographie d'échange d'ions.

L'utilisation de fragments d'ARN pour réduction du poids moléculaire de l'ARN ribosomal améliore sensiblement sa capacité d'augmenter le pouvoir immunogénique des antigènes spécifiques. De nombreuses méthodes classiques, décrites dans la littérature permettent d'y parvenir.

Il est toutefois préférable d'utiliser une méthode enzymatique basée sur l'action de la Ribonucléase pancréatique qui présente l'avantage de respecter les parties en double chaîne de l'ARN ribosomal.

Cette méthode consiste à incuber pendant deux heures à 37 °C la solution d'ARN obtenue avec 5 µg par ml de RNase pancréatique.

L'ARN est ensuite concentré et purifié par chromatographie sur DEAE cellulose comme précédemment. Les fractions contenant de l'ARN sont recueillies par précipitation à l'éthanol.

Une purification complémentaire, mais non indispensable peut être obtenue en sélectionnant les fragments compris entre $5.10^4$ et $2.10^5$ daltons de poids moléculaire, soit par chromatographie de tamisage moléculaire sur Séphadex G200, soit par ultrafiltration sur membrane Diaflo uM 300.

Les préparations d'ARN ainsi obtenues peuvent être contrôlées par les méthodes connues de l'homme de l'art, notamment par la détermination du poids moléculaire:

Détermination du poids moléculaire des préparations

1. pour les ARN non-hydrolysés, par chromatographie de tamisage moléculaire sur Biogel A 1,5m et par ultracentrifugation en gradient de densité.

2. pour les fragments d'ARN, par chromatrographie de tamisage moléculaire sur Séphadex G200 et par électrophorès sur gradient de gels d'acrylamide.

Résultats analytiques moyens

La détermination des poids moléculaires par les différentes méthodes indiquées donne les résultats suivants:

1. pour l'ARN non-hydrolysé, les 2 molécules classiques 16S et 23S sont retrouvées avec des PM respectifs voisins de $5.10^5$ et $1.10^6$ daltons.

2. pour les fragments d'ARN, leur PM moyen se situe entre $5.10^4$ et $2.10^5$ daltons.

La composition physico-chimique des deux types de préparations est pratiquement la même: Elles contiennent au moins 95% d'ARN, mois de 0,25% d'ADN, moins de 3% de protéines, moins de 1% d'hexoses, moins de 0,1% d'hexosamines et moins de 0,0001% de LPS.

Etant donné la nature variée des antigènes spécifiques bactériens, de nombreux procédés peuvent être utilisés pour leur couplage sur les chaînes d'ARN.

Il faut distinguer d'une façon plus générale deux grands groupes d'antigènes:

- les antigènes peptidiques
- les antigènes polysaccharidiques.

Les antigènes peptidiques

De nombreuses techniques de couplage chimique permettent de former des liaisons covalentes entre les groupes aminés des bases nucléiques et les carboxyles ou les fonctions aminées des aminoacides terminaux des antigènes peptidiques. Ces techniques font, par exemple, appel à des réactifs tels que le glutaraldéhyde, les carbodiimides.

L'utilisation en particulier de carbodiimides de structure générale RN = C = NR permet de réaliser une liaison amide entre l'amine d'une des bases de l'ARN et le carboxyle d'un aminoacide. Selon le schéma suivant:

$$NH_2\text{-X-COOR} + R'NH\text{-Y-COOH} \xrightarrow{+\ RN = C = NR}$$

$$R'NH\text{-Y-CO-NH-X-COOR} + H_2O$$

Le carbodiimide peut être, par exemple, le: 1-éthyle-3-(3-diméthylamino propyl) carbodiimide hydrochloride (EDC).

Les conditions opératoires peuvent être les suivantes:

Préparer une solution aqueuse d'ARN à 5 mg d'ARN par ml, ajuster le pH à 4,5 puis ajouter à cette solution 2 mg/ml de carbodiimide. Ajouter à cette préparation une solution de l'antigène peptidique au même pH dans les proportions désirées.

Le mélange est agité une nuit à température ambiante.

Le complexe est précipité par addition de 2 volumes d'éthanol froid en présence de $CH_3COONa$ 0,2M puis lavé par de l'alcool à 70% contenant $CH_2COONa$ 0,2 m.

Le complexe est ensuite dissous dans un tampon convenable, stérilisé par filtration sur membrane 0,2 µ et lyophilisé.

A côté de cette méthode chimique, des complexes peptides-ARN peuvent être également obtenus par irradiation ultraviolette de solution contenant l'ARN et le peptide. Dans ces conditions, des liaisons covalentes sont également obtenues. La longueur d'onde doit être alors comprise entre 223 et 290 nm.

Les antigènes polysaccharidiques

Les ARN ribosomaux possèdent une grande affinité pour les structures polysaccharidiques. Cette propriété peut être exploitée pour réaliser des complexes ARN-antigènes polysaccharidiques.

Les structures secondaire et tertiaire de l'ARN très repliées masquent une grande partie de sites de couplage. Cette structure, après élimination des protéines ribosomales, reste maintenue par

l'action de certains cations divalents comme le magnésium.

Un procédé consiste donc à complexer l'ion magnésium à l'aide d'un agent chelatant, à mettre en présence l'ARN ainsi relâché avec l'antigène spécifique puis à ajouter ensuite des ions magnésium afin d'obtenir un complexe selon l'invention.

Un mode opératoire plus détaillé sera décrit dans les exemples ci-aprés.

Par complexe on entend dans la présente description une molécule présentant une unité qui peut être caractérisée par les méthodes ci-dessous.

Une des méthodes permettant le mieux de caractériser les complexes ainsi préparés est l'ultracentrifugation en gradient de densité.

Sur des gradients de sucrose de 5 à 20% formés dans les tubes d'un rotor SW de 14 ml on dépose la solution de complexe à analyser ainsi que sur les autres tubes un témoin d'ARN seul et un témoin d'antigène seul.

Après stabilisation des bandes sur le gradient (centrifugation isopicnique) (les conditions de vitesse et de durée de la centrifugation sont adaptées pour chaque cas en fonction des poids moléculaires relatifs de l'ARN et de l'antigène), les tubes sont recueillis et les gradients élués au moyen d'une pompe et d'une solution de sucrose à 35%.

L'éluat est fractionné sur un collecteur de fractions et la densité optique de chaque fraction est mesurée à 260 nm. Les courbes de densité optique à 260 nm et d'indice de réfraction sont tracées graphiquement. On peut ainsi visualiser le décalage de densité d'équilibre entre l'ARN témoin et l'ARN alourdi par l'antigène.

De plus, la fraction contenant l'ARN couplé est revueillie et dialysée exhaustivement pour éliminer le sucrose du gradient. (La zone correspondante du tube témoin antigène est traitée dans les mêmes conditions pour vérifier que la zone d'équilibrage de l'antigène témoin est différente).

Après dialyse, une analyse physico-chimique est réalisée, qui permet une mesure quantitative et qualitative de la quantité d'antigène lié à l'ARN.

Deux autres méthodes peuvent également être utilisées pour caractériser ces complexes:

1. La chromatographie de tamisage moléculaire

Sur un Séphadex de porosité appropriée donnant des volumes d'élution différents pour l'ARN et l'antigène témoin, le complexe est chromatographié. La zone présentant une absorption à 260 nm est recueillie et analysée comme précédemment. De plus, la modification du volume d'élution par rapport à l'ARN témoin caractérise l'augmentation du poids moléculaire du complexe.

2. L'électrophorèse sur gel de polyacrylamide en gradients

Sur des plaques on forme des gradients d'acrylamide de 4 à 30% par un procédé classique, puis on dépose l'ARN témoin, l'antigène témoin et le complexe correspondant. Après une nuit de migration, l'électrophorèse est arrêtée et révélée. Les distances de migration sont alors directememt proportionnelles au poids moléculaire des substances et on observe, pour le complexe, une distance de migration correspondant à un poids moléculaire supérieur à celui de l'ARN témoin.

La présente invention concerne également des vaccins contenant à titre de principe actif au moins un complexe tel que défini précédemment.

Les vaccins selon la présente invention peuvent être préparés par tout procédé connu, en particulier sous forme de solution aqueuse, les complexes selon l'invention étant solubles dans l'eau.

Il est possibel évidemment d'utiliser d'autres supports ou additifs compatibles avec l'utilisation en médecine.

Ces vaccins peuvent être administrés également par une voie quelconque qui dépend essentiellement de l'action recherchée et de l'état du patient; ainsi les vaccins peuvent être présentés sous forme d'aérosol ou sous forme injectable en particulier par voie sous-cutanée.

Les dosages journaliers et leur fréquence dépendent également beaucoup de l'affection à prévenir et de l'état du patient, mais si un surdosage n'apporte souvent aucun avantage supplémentaire il ne présente aucun risque compte tenu de la très faible toxicité de ces vaccins.

A titre indicatif et non limitatif on donne ci-après un certain nombre d'exemples de vaccins comprenant des antigènes spécifiques complexés à des ARN ou à des fragments d'ARN ribosomaux.

Exemple 1

Complex ARN Antigène capsulaire d'Hémophilus influenzae comprenant:

| | |
|---|---|
| ARN ribosomal de Klebsiella pneumoniae: | 10 µg |
| Polysaccharide capsulaire d'Hémophilus influenzae b: | 0,5 µg |

Exemple 2

Vaccin pneumococcique composé des complexes suivants:

| | |
|---|---|
| Streptococcus pneumoniae type I | 5 µg d'ARN et 0,2 µg de PS capsulaire |
| Streptococcus pneumoniae type II | 5 µg d'ARN et 0,2 µg de PS capsulaire |
| Streptococcus pneumoniae type III | 5 µg d'ARN et 0,2 µg de PS capsulaire |

Exemple 3

Vaccin bronchique:

| | |
|---|---|
| Klebsiella pneumoniae type I | 5 µg d'ARN et 0,2 µg de PS capsulaire |
| Streptococcus pneumoniae type I | 5 µg d'ARN et 0,2 µg de PS capsulaire |
| Streptococcus pyogènes AR type 12 | 5 µg d'ARN et 0,2 µg d'acide teichoïque |
| Hémophilus influenzae type a | 5 µg d'ARN et 0,2 µg de PS capsulaire |

Exemple 4

Vaccin intestinal

| | |
|---|---|
| Salmonella typhimurium | 3 µg d'ARN et 0,1 µg de proteines de membrane |
| Escherichia coli | 3 µg d'ARN et 0,1 µg de protéines K |
| Shigella dysenteriae | 3 µg d'ARN et 0,1 µg d'antigène 0 |
| Staphylococcus auréus | 3 µg d'ARN et 0,1 µg d'acide teichoïque |

Afin de préciser certaines caractéristiques et avantages de la présente invention les exemples suivants décrivent en détail la préparation de la composition selon l'Exemple 2.

Exemple 5

Séparation des capsules et du contenu cellulaire

La biomasse bactérienne de Streptococcus pneumoniae I utilisée pour la préparation de l'ARN ribosomal et des PS capsulaires est obtenue par un procédé classique de fermentation industrielle. La seule particularité des fermentations destinées à la préparation des ARN ribosomaux réside dans la centrifugation des cultures alors qu'elles sont en pleine phase de croissance exponentielle et à basse température, afin de conserver aux cellules une activité maximale de synthèse ribosomale.

Le concentrat bactérien est lavé par remise en suspension dans du sérum physiologique et centrifugation. La biomasse est soumise à un contrôle de qualité bactériologique, puis stockée congelée à basse température.

Pour obtenir les polysaccharides capsulaires, les cellules lavées sont dispersées dans un tampon tris-HCl 0,01 M, pH 7, contenant NaCl 0,10 M et pH 7, contenant NaCl 0,10 M et $MgCl_2$ 0,01 M. La température de la suspension est portée à 40 °C et les capsules sont séparées par homogénéisation quelques minutes dans un warring blendor à cette température.

Après refroidissement, les cellules décapsulées sont séparées par une centrifugation de 30 minutes à 30 000 g et 4 °C.

Le culot est séparé pour la préparation des ribosomes et de l'ARN ribosomal. Le surnageant contenant les capsules est recueilli; son traitement sera décrit dans l'exemple.

Afin de recueillir le contenu intracellulaire, le culot est remis en suspension dans du tampon tris- HCl pH 7,2 contenant $MgCl_2$ et NaCl puis la suspension est soumise à une désintégration cellulaire sur un appareil DYNO-MILL à micro billes de verre, réfrigéré.

Exemple 6

Préparation de la fraction ribosomale

Les cellules non broyées et les débris cellulaires sont éliminés par deux centrifugations successives à 10 000 puis à 30 000 g à +4 °C.

Le surnageant clair obtenu est traité une heure à 37 °C par 5 µg/ml de D Nase puis acidifié à pH 5. Le précipité de protéines formé à ce pH est éliminé par centrifugation à 30 000 g et +4 °C.

Les ribosomes sont précipités du surnageant par acidification à pH 4 et recueillis par centrifugation à 10 000 g et +4 °C.

Les protéines non ribosomales absorbées sont donc éliminées par un lavage des ribosomes 30 minutes à 25 °C dans une solution de SDS à 0,25% (Sodium dodécyl sulfate) dans du tampon tris-HGI pH 7,5 contenant $MGCl_2$ et NaCl.

Les ribosomes lavés sont précipités de cette solution par l'addition de 0,7 volume d'alcool éthylique glacé. Le précipité de ribosomes est recueilli par centrifugation puis repris dans du tempon tris-HCl pH 7,2 contenant NaCl.

La solution est clarifiée par centrifugation à 30 000 g et le surnageant contenant les ribosomes est conservé pour la préparation de l'ARN.

Exemple 7

Préparation de l'ARN ribosomal

La plus grande partie des protéines ribosomales est éliminée de l'ARN au moyen d'un traitement par le SDS à chaud. A la suspension de ribosomes précédentes, on ajoute du SDS pour avoir une concentration finale de 1%. La solution est alors portée rapidement à 80 °C et maintenue 5 minutes à cette température avec une agitation énergique. Après un refroidissement rapide, l'ARN est précipité par acidification à pH 4 avec HCl dilué, dans ces conditions l'ARN 5S n'est pas précipité, seuls les ARN 16S et 23S précipitent. Le précipité d'ARN est recueilli par centrifugation, puis lavé par une solution à 70% d'étanol contenant 0,2 M d'acétate de sodium à pH 7,8 pour éliminer le SDS résiduel. L'ARN ainsi lavé et transformé en sel de sodium est repris dans du tampon tris-HCl pH 7,8 et contenant NaCl.

A ce stade, 5 à 10% de protéines peuvent encore contaminer l'ARN. Leur élimination est obtenue par un traitement de 1 heure à 37 °C avec de la pronase.

L'ARN est précipité de cette solution par addition lente d'un demi-volume d'une solution aqueuse à 5% de Cétavlon. (Bromure de Cétyltriméthylammonium et recueilli aussitôt par centrifugation).

Le précipité est ensuite lavé plusieurs fois par dispersion et centrifugation dans une solution à 70% d'éthanol contenant 0,2 M d'acétate de sodium à pH 7,8 ce qui élimine le cétavlon résiduel.

Le résidu d'ARN est repris dans du tampon tris-HCl 0,05 M pH 7,2 puis soumis à une dernière purification par chromatographie d'échange d'ions sur une colonne de DEAE cellulose. L'ARN retenu sur cette colonne est élué après lavage par un gradient de NaCl (0 → 0,5 M) dans le même tampon. La fraction contenant l'ARN purifié est recueillie, puis précipitée par addition de 2 volumes d'éthanol glacé. L'ARN est recueilli par centrifugation et repris dans un volume minimum de tampon tris-HCl 0,01 M pH 7,5.

Exemple 8

Le surnageant contenant les capsules obtenu dans l'Exemple 5 est traité de la façon suivante.

Les polysaccharides capsulaires sont tout d'abord précipités à partir du surnageant par une addition lente d'une solution aqueuse de chlorure de cétylpyridinium à 2%, jusqu'en fin de floculation. Après une heure de repos à 4 °C, le précipité de polysaccharide est recueilli par centrifugation et le surnageant écarté.

Le complexe polysaccharide-chlorure de cétylpyridinium précipité est dissocié dans du chlorure de sodium 2 M, dans ces conditions le polysaccharide passe en solution alors que le chlorure de cétylpyridinium est éliminé par centrifugation et le surnageant conservé.

Trois volumes d'alcool éthylique froid sont ajoutés au surnageant pour précipiter le polysaccharide qui est recueilli par centrifugation après une heure de repos à 4 °C.

Le polysaccharide précipité est lavé par dispersion dans un mélange eau-éthanol (30 : 70 v/v) contenant $CH_3COO$ Na 0,1 M, pendant 30 minutes à température ambiante. La solution de lavage est éliminée par centrifugation et le culot est repris dans une solution aqueuse 0,1 M de $CH_3COO$ Na.

Afin d'éliminer les protéines pouvant encore contaminer la préparation à ce stade, il est procédé à une extraction 10 minutes à 65 °C par un volume de phénol à 90% dans l'acétate de sodium 1 M. Après agitation vive, 10 minutes à 65 °C, la mélange est refroidi rapidement dans un bain de glace et les deux phases sont séparées par centrifugation 5 minutes à 10 000 g et 0 °C. La phase aqueuse supérieure est prélevée délicatement. Trois volumes d'alcool éthylique froid sont ajoutés à la phase aqueuse et le polysaccharide est laissé en précipitation 1 heure à 4 °C.

Le précipité est recueilli par centrifugation puis dispersé dans un mélange eau-éthanol (30 : 70 v/v) contenant $CH_3COO$ Na 0,1 M pendant 30 minutes à température ambiante. Le précipité lavé est recueilli par centrifugation et repris dans l'eau distillée puis stérilisé par filtration sur membrane 0;22 µ et lyophilisé.

Exemple 9

Préparation du complexe

On ajoute à une solution aqueuse à 5 mg d'ARN/ml préparé par le procédé de l'Exemple 7 une solution 0,001 M d'acide éthylène diamine-tétraacétique (EDTA) qui complexe les ions magnésium qui sont ensuite éliminés par dialyse.

La structure de l'ARN se trouve ainsi «relâchée» démasquant un nombre maximum de sites de couplage. L'antigène polysaccharidique obtenu à l'Exemple 8 est alors dissous à 0,2 mg/ml dans la solution d'ARN et le mélange est agité pendant une nuit à 4 °C. Après ce temps d'agitation, du chlorure de magnésium est alors ajouté à la solution pour avoir une molarité finale de 0,01 M et l'agitation est poursuivie pendant deux heures.

Le complexe est alors précipité en présence de $CH_3COONa$ 0,2 M par addition de 2 volumes d'éthanol froid.

La précipité est lavé par l'éthanol à 70% contenant $CH_3COONa$ 0,2 M puis repris dans un tampon convenable, stérilisé par filtration sur membrane 0,2 µ et lyophilisé.

On prépare de la même façon le complexe correspondant à S. pneumoniae type II et S. pneumoniae type III.

Exemple 10

Etude de la protection de la souris par la formulation N° 2, contre un challenge par la souche virulente de Streptococcus pneumoniae type I

Animaux utilisés
souris Balb/c

Formule N° 2
Streptococcus pneumoniae Type I:
5 µg d'ARN + 0,2 µg de PS
Streptococcus pneumoniae Type II:
5 µg d'ARN + 0,2 µg de PS
Streptococcus pneumoniae Type III:
5 µg d'ARN + 0,2 µg de PS

Méthodologie:

- A – 20 souris traitées 2 fois par 1,25 µg de mélange en 15 jours, par voie sous-cutanée.
- A' – 20 souris témoins traitées 2 fois par 0,2 ml d'eau salée en 15 jours par voie sous-cutanée.
- B – 20 souris traitées 2 fois par 0,6 µg du mélange en 8 jours, par voie sous-cutanée.
- B' – 20 souris témoins traitées 2 fois par 0,2 ml d'eau salée en 8 jours par voie sous-cutanée.

Le challenge a lieu le 6ème jour après le dernier traitement avec la souche virulente de Streptococcus pneumoniae Type I.

Résultats:

Groupe de souris A/A'

1. Challenge par 50 bactéries (Streptococcus pneumoniae I) par voie intrapéritonéale.
   → Protection + + très significative, 20 jours après le challenge.
   (groupe A – 20 animaux survivants
   (groupe A' témoins – 12 animaux survivants
2. Challenge par 5 bactéries (Streptococcus pneumoniae I) par voie intrapéritonéale.
   → Protection + + très significative, 20 jours après le challenge.
   (groupe A – 20 animaux survivants
   (groupe A' témoins – 12 animaux survivants.
3. Challenge par 1 bactérie (Streptococcus pneumoniae I) par voie intrapéritonéale.
   → Pas d'infection chez les animaux traités ou vaccinés.

Groupe de souris B/B′

1. Challenge par 100 bactéries (Streptococcus pneumoniae I) par voie intrapéritonéale.
   → Protection ++ très significatife, 2 jours après le challenge.
   (groupe B – 20 animaux survivants
   (groupe B′ témoins – 0 animal survivant.
2. Challenge par 10 bactéries (Streptococcus pneumoniae I) par voie intrapéritonéale.
   → Protection ++ très significative, 2 jours après le challenge.
   (groupe B – 20 animaux survivants
   (groupe B′ témoins – 0 animal survivant.

Pour les deux challenges, tous les animaux traités survivent 20 jours après l'administration de germes virulents alors que les animaux témoins sont tous morts 2 jours après.

Recherche des anticorps spécifiques par immunoélectrodiffusion diriges contre les trois antigenes dont sont issus les A.R.N. et les P. S. capsulaires

| Souris femelles $OF_1$ | Streptococcus pneumoniae Type I (Hauteur des «Rockets» en mm) | | Streptococcus pneumoniae Type II (Hauteur des «Rockets» en mm) | | Streptococcus pneumoniae Type III (Hauteur des «Rockets» en mm) | |
|---|---|---|---|---|---|---|
| | Avant traitement | Après traitement | Avant traitement | Après traitement | Avant traitement | Après traitement |
| 1 | 10 | 20 | 9 | 13 | 8 | 15 |
| 2 | 8 | 20 | 8 | 15 | 10 | 15 |
| 3 | 8 | 18 | 0 | 16 | 10 | 14 |
| 4 | 6 | 18 | 0 | 15 | 6 | 12 |
| 5 | 4 | 17 | 4 | 16 | 5 | 16 |
| 6 | 10 | 20 | 12 | 20 | 12 | 18 |
| 7 | 10 | 19 | 0 | 16 | 7 | 14 |
| 8 | 10 | 16 | 7 | 12 | 7 | 16 |
| 9 | 8 | 17 | 7 | 16 | 0 | 12 |
| 10 | 10 | 15 | 7 | 12 | 8 | 16 |

Protocole de vaccination avec la formulation de l'exemple n° 2, 5 injections en 2 semaines.
¼ de dose par injection, soit 1,25 µg d'A.R.N. + 0,05 µg de P.S. de chaque type par animal.

**Revendications**

1. Complexe vaccinal caractérisé en ce qu'il est constitué par des ARN ribosomaux bactériens ou des fragments d'ARN ribosomaux bactériens sur lesquels on a couplé de 1 à 5% en poids d'un antigène spécifique de sérotype bactérien.

2. Complexe selon la revendication 1, caractérisé en ce que les ARN et l'antigène spécifique proviennent des mêmes souches bactériennes.

3. Complexe selon la revendication 1, caractérisé en ce que les ARN et l'antigène spécifique proviennent de souches bartériennes différentes.

4. Complexe selon l'une des revendications 1 à 3, caractérisé en ce que l'antigène spécifique est choisi parmi:
- les polysaccharides capsulaires,
- les lipopolysaccharides membranaires des bactéries gram négatives,
- les protéines membranaires spécifiques,
- les acides teichoïques et lipotéichoïques des bactéries gram négatives.

5. Complexe selon la revendication 4, caractérisé en ce que l'antigène spécifique est choisi parmi les polysaccharides capsulaires de:
- Klebsiella pneumoniae,
- Streptococcus pneumoniae,
- Hemophilus influenzae,
- Escherichia coli.

6. Complexe selon la revendication 4, caractérisé en ce que l'antigène spécifique est choisi parmi les lipopolysaccharides membranaires de:
- Klebsiella pneumoniae,
- Serratia marcenscens,
- Escherichia coli,
- Neisseria meningitidis,
- Salmonella typhimurium.

7. Complexe selon la revendication 4, caractérisé en ce que l'antigène spécifique est choisi parmi les protéines membranaires spécifiques de:
- Escherichia coli,
- Serratia marcescens,
- Salmonella typhimurium,
- Streptococcus pyogenes.

8. Complexe selon la revendication 4, caractérisé en ce que l'antigène spécifique est choisi parmi les acides teichoïques et lipotéichoïques de:
- Streptocoques,
- Staphylocoques,
- Lactobacilles.

9. Complexe selon l'une des revendications 1 et 3 à 8, caractérisé en ce que les ARN ribosomaux sont extraits des ribosomes de:
- Streptococcus pneumoniae,
- Streptococcus pyogenes,
- Staphylococcus aureus,
- Klebsiella pneumoniae,
- Serratia marcescens,
- Escherichia coli,
- Salmonella typhimurium,
- Corynebacterium,
- Mycobacterium.

10. Complexe selon l'une des revendications 1 à 9, caractérisé en ce que les fragments d'ARN ribosomaux bactériens ont un poids moléculaire compris entre $3 \times 10^4$ et $2 \times 10^5$ daltons.

11. Complexe selon la revendication 10, caractérisé en ce que les fragments d'ARN ribosomaux bactériens ont un poids moléculaire compris entre 30 000 et 50 000.

12. Complexe selon l'une des revendications 1 à 11, caractérisé en ce que le couplage est réalisé par des liaisons covalentes.

13. Vaccin caractérisé en ce qu'il comporte à titre de principe actif un complexe selon l'une des revendications 1 à 12.

## Claims

1. Vaccine complex, characterised in that it consists of bacterial ribosomal RNA, or fragments of bacterial ribosomal RNA, to which there has been coupled from 1 to 5% by weight of a specific antigen of bacterial serotype.

2. Complex according to Claim 1, characterised in that the RNA and the specific antigen originate from the same bacterial strain.

3. Complex according to Claim 1, characterised in that the RNA and the specific antigen originate from different bacterial strains.

4. Complex according to one of Claims 1 to 3, characterised in that the specific antigen is chosen from:
- capsular polysaccharides,
- membrane lipopolysaccharides of Gram-negative bacteria,
- specific membrane proteins
- teichoic and lipoteichoic acids of Gram-negative bacteria.

5. Complex according to Claim 4, characterised in that the specific antigen is chosen from the capsular polysaccharides of:
- Klebsiella pneumoniae,
- Streptococcus pneumoniae,
- Hemophilus influenzae,
- Escherichia coli.

6. Complex according to Claim 4, characterised in that the specific antigen is chosen from the membrane lipopolysaccharides of:
- Klebsiella pneumoniae,
- Serratia marcenscens,
- Escherichia coli,
- Neisseria meningitidis,
- Salmonella typhimurium.

7. Complex according to Claim 4, characterised in that the specific antigen is chosen from the specific membrane proteins of:
- Escherichia coli,
- Serratia marcescens,
- Salmonella typhimurium,
- Streptococcus pyogenes.

8. Complex according to Claim 4, characterised in that the specific antigen is chosen from the teichoic and lipoteichoic acids of:
- Streptococci,
- Staphylococci,
- Lactobacilli.

9. Complex according to one of Claims 1 and 3 to 8, characterised in that the ribosomal RNA is extracted from the ribosomes of:
- Streptococcus pneumoniae,
- Streptococcus pyogenes,
- Staphylococcus aureus,
- Klebsiella pneumoniae,
- Serratia marcescens,
- Escherichia coli,
- Salmonella typhimurium,
- Corynebacterium,
- Mycobacterium.

10. Complex according to one of Claims 1 to 9, characterised in that the fragments of bacterial ribosomal RNA have a molecular weight of between $3 \times 10^4$ and $2 \times 10^5$ daltons.

11. Complex according to Claim 10, characterised in that the fragments of bacterial ribosomal RNA have a molecular weight of between 30,000 and 50,000.

12. Complex according to one of Claims 1 to 11, characterised in that the coupling is carried out by means of covalent bonds.

13. Vaccine, characterised in that it incorporates by way of an active principle a complex according to one of Claims 1 to 12.

## Patentansprüche

1. Impfkomplex, dadurch gekennzeichnet, dass er von ribosomaler RNA aus Bakterien oder von ribosomalen RNA-Fragmenten aus Bakterien gebildet wird, an die 1 bis 5 Gew.-% eines spezifischen Antigens eines bakteriellen Sterotyps gekoppelt sind.

2. Komplex nach Anspruch 1, dadurch gekennzeichnet, dass die RNA und das spezifische Antigen aus den gleichen Bakterienstämmen stammen.

3. Komplex nach Anspruch 1, dadurch gekennzeichnet, dass die RNA und das spezifische Antigen aus verschiedenen Bakterienstämmen stammen.

4. Komplex nach einem der Ansprüche 1 bis 3, dadurch gekennzeichnet, dass das spezifische Antigen aus:
- Kapselpolysacchariden,
- Membranlipopolysacchariden gramnegativer Bakterien,
- spezifischen Membranproteinen,

– Teichon- und Lipoteichonsäuren gramnegativer Bakterien ausgewählt wird.

5. Komplex nach Anspruch 4, dadurch gekennzeichnet, dass das spezifische Antigen aus den Kapselpolysacchariden von:
– Klebsiella pneumoniae,
– Streptococcus pneumoniae,
– Hemophilus influenzae,
– Escherichia coli ausgewählt wird.

6. Komplex nach Anspruch 4, dadurch gekennzeichnet, dass das spezifische Antigen aus den Membranlipopolysacchariden von:
– Klebsiella pneumoniae,
– Serratia marcescens,
– Escherichia coli,
– Neisseria meningitidis,
– Salmonella typhimurium ausgewählt wird.

7. Komplex nach Anspruch 4, dadurch gekennzeichnet, dass das spezifische Antigen aus den spezifischen Membranproteinen von:
– Escherichia coli,
– Serratia marcescens,
– Salmonella typhimurium,
– Streptococcus pyogenes ausgewählt wird.

8. Komplex nach Anspruch 4, dadurch gekennzeichnet, dass das spezifische Antigen aus den Teichon- und Lipoteichonsäuren von:
– Streptokokken,
– Staphylokokken,
– Lactobazillen ausgewählt wird.

9. Komplex nach einem der Ansprüche 1 und 3 bis 8, dadurch gekennzeichnet, dass die ribosomalen RNA aus Ribosomen von:
– Streptococcus pneumoniae,
– Streptococcus pyogenes,
– Staphylococcus aureus,
– Klebsiella pneumoniae,
– Serratia marcescens,
– Escherichia coli,
– Salmonella typhimurium,
– Corynebacterium,
– Myobacterium extrahiert werden.

10. Komplex nach einem der Ansprüche 1 bis 9, dadurch gekennzeichnet, dass die ribosomalen RNA-Fragmente aus Bakterien ein Molekulargewicht zwischen $3 \times 10^4$ und $2 \times 10^5$ Dalton haben.

11. Komplex nach Anspruch 10, dadurch gekennzeichnet, dass die ribosomalen RNA-Fragmente aus Bakterien ein Molekulargewicht zwischen 30 000 und 50 000 haben.

12. Komplex nach einem der Ansprüche 1 bis 11, dadurch gekennzeichnet, dass die Koppelung durch kovalente Bindungen erfolgt.

13. Impfstoff, dadurch gekennzeichnet, dass er als aktiven Bestandteil einen Komplex nach einem der Ansprüche 1 bis 12 enthält.